# EUROPEAN PATENT SPECIFICATION

(11) **EP 1 073 452 B1**
(45) Date of publication and mention of the grant of the patent: **02.01.2003**
(21) Application number: 99921558.5
(22) Date of filing: 30.04.1999
(51) Int. Cl.: A61K 38/08, A61P 1/16

(54) **USE OF DELTORPHIN FOR TREATING CYTOKINE MEDIATED HEPATIC INJURY**
VERWENDUNG VON DELTORPHIN ZUR BEHANDLUNG VON ZYTOKIN-VERMITTELTER LEBERSCHÄDIGUNG
UTILISATION DE LA DELTORPHINE POUR LE TRAITEMENT D'UNE MALADIE HEPATIQUE LIEE A LA CYTOKINE

(30) Priority: 01.05.1998 US 71255
(43) Date of publication of application: 07.02.2001
(73) Proprietor: University of Kentucky Research Foundation, Lexington, KY 40506-0032 (US); ZymoGenetics, Seattle, WA 98102 (US)
(72) Inventor: OELTGEN, Peter, R., Winchester, KY 40391 (US); MCCLAIN, Craig, I., Lexington, KY 40503 (US); BARVE, Shirish, Lexington, KY 40509 (US); BISHOP, Paul, D., Fall City, WA 98024 (US)
(74) Representative: Findlay, Alice Rosemary
(86) International application number: US9909457
(87) International publication number: WO99056767

(56) References cited:
- MORGAN EL: "Regulation of human B lymphocyte activation by opioid peptide hormones - Inhibition of IgG production by opioid receptor class (gamma-, kappa-, and delta-) selective agonists" J NEUROIMMUNOLOGY, vol. 65, no. 1, 1996, pages 21-30, XP000857152
- MALAGUARNERA M ET AL: "Elevation of interleukin 6 levels in patients with chronic hepatitis due to hepatitis C virus" J GASTROENTEROL, vol. 32, 1997, pages 211-215, XP000857431
- HOUSE R V ET AL: "A comparative study of immunomodulation produced by in vitro exposure to delta opioid receptor agonist peptides." PEPTIDES, (1996) 17 (1) 75-81. , XP000857392
- THORNTON JR ET AL: "Opioid peptides and primary biliary cirrhosis" BMJ, vol. 297, no. 6662, 1988, pages 1501-4, XP000857137
- THOMAS SA ET AL: "Structure-activity relationships of a series of [D-Ala2]deltorphin I and II analogues; in vitro blood-brain barrier permeability and stability" J PHARMACOL EXP THER, vol. 281, no. 2, 1997, pages 817-825, XP000857782

### SEQUENCE LISTING

(1) GENERAL INFORMATION:
   (i) APPLICANTS: OELTGEN, PETER R.
      McCLAIN, CRAIG J.
      BARVE, SHIRISH
   (ii) TITLE OF INVENTION: METHOD FOR TREATING CYTOKINE
      MEDIATED HEPATIC INJURY
   (iii) NUMBER OF SEQUENCES: 2
   (iv) CORRESPONDENCE ADDRESS:
      (A) ADDRESSEE: WOOD, HERRON & EVANS
      (B) STREET: 441 VINE STREET, SUITE 2700
      (C) CITY: CINCINNATI
      (D) STATE: OH
      (E) COUNTRY: U.S.
      (F) ZIP: 45202-2917
   (v) COMPUTER READABLE FORM:
      (A) MEDIUM TYPE: 3.5 DS, HD Diskette
      (B) COMPUTER: IBM Compatible
      (C) OPERATING SYSTEM: Windows 95
      (D) SOFTWARE: FastSEQ for Windows Version 2.0b
   (vi) CURRENT APPLICATION DATA:
      (A) APPLICATION NUMBER: Unknown
      (B) FILING DATE: 01-MAY-1998
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME: Lyman, Beverly A
      (B) REGISTRATION NUMBER: P-41,961
      (C) REFERENCE/DOCKET NUMBER: ZYM-02
   (ix) TELECOMMUNICATION INFORMATION:
      (A) TELEPHONE: 513-241-2324
      (B) TELEFAX: 513-421-7269
      (C) TELEX:
(2) INFORMATION FOR SEQ ID NO:1:
   (i) SEQUENCE CHARACTERISTICS:
      (A) LENGTH: 7 amino acids
      (B) TYPE: amino acid
      (C) STRANDEDNESS:
      (D) TOPOLOGY:
   (ii) MOLECULE TYPE: peptide
   (vi) ORIGINAL SOURCE:
      (A) ORGANISM:
   (xi) SEQUENCE DESCRIPTION: SEQ ID NO:1:
   Tyr Ala Phe Asp Val Val Gly
    1               5
(2) INFORMATION FOR SEQ ID NO:2:
   (i) SEQUENCE CHARACTERISTICS:
      (A) LENGTH: 7 amino acids
      (B) TYPE: amino acid
      (C) STRANDEDNESS:
      (D) TOPOLOGY:
   (ii) MOLECULE TYPE: peptide
   (xi) SEQUENCE DESCRIPTION: SEQ ID NO:2:
   Tyr Ala Phe Glu Val Val Gly
    1               5

## Description

### Field of the Invention

The invention relates to the use of deltorphins to attenuate or prevent cytokine mediated hepatic injury.

### Background

Hepatic injury can be caused by a number of different agents including viruses such as Hepatitis A, B, C, D and E, both gram positive and gram negative bacteria, chemical agents such as ethanol, carbon tetrachloride and lead, and by physical trauma resulting in ischemia (ischemic hepatitis) injuries as can occur in right-sided congestive heart failure. It is now believed that all of these types of hepatic injury are caused at least in part by the liver's inflammatory or cytokine response to these agents. The inflammatory response of the liver results in the overexpression of a cascade of inflammatory/acute phase cytokines, such as interleukin-1 (IL-1), tumor necrosis factor (TNF), IL-6, IL-8 and transforming growth factor beta (TGFβ). It is now believed that it is the cascade of these cytokines which is the ultimate cause of much of the hepatic injury resulting from these agents. Thus, there is a need for a therapeutic agent which can be useful in alleviating or modulating the inflammatory response associated with liver disease or injury.

### Summary of the Invention

The present invention fills this need by providing the use of a deltorphin for the manufacture of a mediament for treating or preventing a cytokine mediated hepatic injury in a mammal. The hepatic injury can be an acute inflammatory reaction, as a result of a viral or bacterial infection or a chemical agent such as ethanol, lead, carbon tetrachloride or acetaminophen, or from trauma resulting in ischemia or reperfusion injury in the liver.

The present invention is also directed to the use of a deltorphin for the manufacture of a mediament for treating a viral or bacterial infection-related hepatic damage in a mammal.

The present invention is also directed to the use of a deltorphin for the manufacture of a mediament for treating alcohol induced liver injury in a mammal. Preferably, the deltorphin is administrable in a pharmaceutical composition at a dosage of from about 0.5 mg/kg to about 20 mg/kg, or alternatively lower doses from about 1 µg/kg to about 1000 µg/kg of deltorphin per body weight of the mammal.

Preferably, the mammal is a human.

### Detailed Description

Deltorphins are endogenous linear heptapeptides isolated from skin extracts of the South American frog *Phyllomedusa bicolor.* These may be further divided into deltorphin I SEQ ID NO:1 and deltorphin II SEQ ID NO:2 depending on their amino acid sequence. Deltorphin I SEQ ID NO:1 has the amino acid sequence Tyr-Ala-Phe-Asp-Val-Val-Gly-NH₂ with alanine as either the D- or L- isomer. Deltorphin II SEQ ID NO:2 has the amino acid sequence Tyr-Ala-Phe-Glu-Val-Val-Gly-NH₂ with alanine as either the D- or L- isomer. Either deltorphin I SEQ ID NO:1, deltorphin II SEQ ID NO:2 or a combination of deltorphin I SEQ ID NO:1 and II SEQ ID NO:2 may be used in the invention. Deltorphins may be obtained from frog skin extracts, or they may be purchased from a vendor such as Peninsula Laboratories, Inc. (Belmont, CA) or may be synthesized using a peptide synthesizer such as the type available from Applied Biosystems.

Deltorphins are to be administered to a mammal to modulate cytokine activation by blocking one or more steps in the cytokine cascade. Deltorphins may be formulated for administration in an aqueous based liquid such as phosphate buffered saline to form an emulsion, or they may be formulated in an organic liquid such as dimethylsulfoxide to form a solution, or they may be formulated in a suspension. The solution, suspension or emulsion is administrable by any route, but it preferably is administrable parenterally such as by intravenous, intramuscular, intradermal or intraperitoneal injections. A preferred dose is in the range of about 0.5-20 mg, or alternatively lower doses of about 1-1000 µg of deltorphin per kg of body weight of the mammal. The time of administration of the deltorphin is preferably prior to initiation of cytokine activation. However, the deltorphin is administrable concurrently with another agent that induces cytokine activation or even subsequent to an agent that induces cytokine activation and still produce a protective effect.

Deltorphin administration should be continued on a daily basis until hepatic function returns to normal and is maintained at normal levels, preferably for at least one to two days. Hepatic injury can be determined by elevated levels of hepatic enzymes, as well as by depressed albumin levels (less than about 35 g/liter). Hepatic function is routinely monitored by quantitating serum levels of hepatic enzymes such as alanine aminotransferase (ALT) (normal < 35 U/L). aspartate aminotransferase (AST) (normal < 30 U/L), alkaline phosphatase (ALP) (normal ≤ 100 U/L) and gamma glutamyltransferase (GGT) (normal ≤ 45 U/L for males, ≤ 30 U/L for females), as well as bilirubin, both conjugated (normal ≤ 0.2 mg/deciliter) and total (normal ≤ 1.0 mg/deciliter) bilirubin. Deltorphin modulation of hepatocyte cytokine activation may be used therapeutically in a variety of hepatic injury processes. As used herein, the term hepatic injury broadly encompasses all types of injury such as hepatic trauma, physical and/or chemical insult, stress, inflammation, toxicity, disease and so on. For example, deltorphins can be used in treating hepatic injury due to alcoholic liver disease, acetaminophen toxicity, cadmium toxicity, lead poisoning, bacteremia due to, for example, *Staphylococcus* species, *Streptococcus* species, *Neisseria* species, *Salmonella* species, *Shigella* species, *Escherichia coli, Clostridium perfringens, Klebsiella* species, *Proteus* species, *Enterobacter* species, *Bacteroides* species, *Brucella* species, *Francisella tularensis, Listeria monocytogenes, Acinetobacter* species, *Streptobacillus moniliformis, Vibrio* species, *Helicobacter pylori, Pseudomonas* species, *Haemophilus* species, *Bordetella pertussis,* viral infections due to, for example, influenza viruses, adenoviruses, paramyxoviruses, rubella viruses, polioviruses, hepatitis viruses, herpesviruses, rabies viruses, human immunodeficiency viruses and papilloma viruses, as well as trauma, ischemia reperfusion injury and metabolic liver disease.

While the specific mechanism of deltorphin action on the modulation of cytokine mediated hepatic injury such as acute inflammatory reactions, trauma and toxin induced biological responses is unknown, deltorphins exhibit a specific and reproducible effect on decreasing hepatotoxicity. This invention will be further appreciated in light of the following example.

### EXAMPLE

Hepatic failure was induced in mice by intraperitoneal injections of a mixture of lipopolysaccharide (LPS) (100 µg/kg) and D-galactosamine (GAL) (700 mg/kg) to form LPS/GAL. LPS is a bacterial endotoxin that is a major stimulus for production of many cytokines, particularly tumor necrosis factor (TNF), thought to be involved in liver injury. GAL is an amino sugar that sensitizes hepatocytes by depleting the base uridine and thus preventing hepatic transcription, and sensitizes animals to normally innocuous amounts of endotoxin or TNF. The LPS/GAL treatment induces fulminate hepatic and multiorgan failure, and serves as a model for septic shock. The major effects of LPS/GAL are elicited by the inflammatory cytokine TNF-α. Hepatocytes sensitized to TNF-α then undergo apoptosis or programmed cell death in response to inflammatory processes.

Mice were treated with deltorphin and were subsequently rendered hepatotoxic by LPS/GAL treatment. An emulsion of deltorphin I SEQ ID NO:1 (Tyr-D-Ala-Phe-Asp-Val-Val-Gly-NH₂) was prepared at a concentration of 80 µg/200 µl in sterile phosphate buffered saline (PBS). An emulsion of DADLE (Tyr-D-Ala-Gly-Phe-D-Leu-enkephalin) was also prepared at a concentration of 80 µg/200 µl in sterile PBS. Male BALB/c HSD mice were treated by intraperitoneal injections according to the following protocol. Control mice (n = 7) received only LPS/GAL (280 µl) at 6.5 h. Deltorphin I SEQ ID NO:1 treated mice (n = 15) received injections of 4 mg/kg deltorphin I SEQ ID NO:1 at 0 h, 2 h, 4 h, 6 h and 7.5 h and received an LPS/GAL injection at 6.5 h. DADLE treated mice (n = 15) received 4 mg/kg DADLE at 0 h, 2 h, 4 h, 6 h and 7.5 h and received an LPS/GAL injection at 6.5 h.

Both control and treated mice were monitored at 15 h, 20 h, 40 h and 65 h after the last injection. The results are shown in the following table.

**TABLE**

| TREATMENT | SURVIVAL | | | |
|---|---|---|---|---|
| | 15 Hours | 20 Hours | 40 Hours | 65 Hours |
| LPS/GAL | 0 | --- | --- | --- |
| DADLE + LPS/GAL | 3 (20%) | 3 (20%) | 3 (20%) | 3 (20%) |
| Deltorphin I SEQ I NO:1 + LPS/GAL | 12 (80%) | 11 (73%) | 11 (73%) | 11 (73%) |

Of the seven mice receiving only LPS/GAL (control group), none survived more than 15 h post-LPS/GAL injection and some died as early as 6 to 8 h post injection. Of the 15 mice treated with DADLE, 20% (3 out of 15) survived for 20 h, 20% (3 out of 15) survived for 40 h and 20% (3 out of 15) survived for 65 h after the last injection. In contrast, of the 15 mice treated with deltorphin I SEQ ID NO:1, 80% (12 out of 15) survived for 15 h, 73% (11 out of 15) survived for 20 h, 73% (11 out of 15) survived for 40 h and 73% (11 out of 15) survived for 65 h after the last injection. The markedly increased survival time for deltorphin I SEQ ID NO:1 treated mice that had been made septic by LPS/GAL treatment was significant compared to mice receiving LPS/GAL only. In contrast, the survival time for DADLE treated mice was not as striking at any time point. These results indicate that treatment with deltorphin I SEQ ID NO:1 prevents acute inflammatory hepatic injury and can reduce lethality in a murine model.

A treatment for attenuating and/or preventing cytokine mediated acute inflammatory, trauma induced and toxin induced hepatic injury is thus disclosed. Deltorphins, administered at a concentration of about 0.5 mg/kg to about 20 mg/kg, or alternatively at lower concentrations of about 1-1000 µg/kg, inhibit hepatic injury and result in decreased lethality of the animal.

It should be understood that the embodiments of the present invention shown and described in the specification are only preferred embodiments of the inventors who are skilled in the art and thus are not limiting in any way. Therefore various changes, modifications or alterations to these embodiments may be made or resorted to without departing from the scope of the following claims.

## Claims

1. The use of a deitorphin for the manufacture of a medicament for the treatment of cytokine mediated hepatic injury.

2. The use of claim 1 wherein said deltorphin is selected from the group consisting of deltorphin I SEQ ID NO: 1, deltorphin II SEQ ID NO: 2 and combinations thereof.

3. The use of claim 1, said deltorphin being in a formulation selected from the group consisting of a solution, an emulsion and a suspension.

4. The use of claim 1, said medicament being for parenteral administration.

5. The use of claim 1, said medicament being formulated for administration at a concentration in the range of about 1 µg/kg to about 20,000 µg/kg.

6. The use of claim 1, said medicament being for the treatment of injury from a chemical toxin selected from the group consisting of ethanol, lead, cadmium, carbon tetrachloride, and acetaminophen.

7. The use of claim 1, said medicament being for the treatment of injury from a bacterial or viral infection.

8. The use of claim 7 wherein the bacterial or viral infection is caused by an organism selected from the group consisting of *Staphylococcus* species, *Streptococcus* species, *Neisseria* species, *Salmonella* species, *Shigella* species, *Escherichia coli, Clostridium perfringens, Klebsiella* species, *Proteus* species, *Enterobacter* species, *Bacteroides* species, *Brucella* species *Francisella tularensis, Listeria monocytogenes, Acinetobacter* species, *Streptobacillus monoiliformis, Vibrio* species, *Helicobacter pylori, Pseudomonas* species, *Haemophilus* species, *Bordetella pertussis*, influenza viruses, adenoviruses, paramyxoviruses, rubella viruses, polioviruses, hepatitis viruses, herpesviruses, rabies viruses, human immunodeficiency viruses andpapilloma viruses.

9. The use of claim 1, said deltorphin being in a pharmaceutically acceptable formulation.

## Patentansprüche

1. Verwendung von Deltorphin zur Herstellung eines Medikaments *zur* Behandlung von zytokinvermittelter Leberschädigung.

2. Verwendung nach Anspruch 1, wobei das Deltorphin ausgewählt ist aus Deltorphin I Seq. Id. Nr. 1, Deltorphin II Seq. Id. Nr. 2 und Kombinationen davon.

3. Verwendung nach Anspruch 1, wobei sich das Deltorphin in einer Formulierung befindet, die ausgewählt ist aus einer Lösung, einer Emulsion und einer Suspension.

4. Verwendung nach Anspruch 1, wobei das Medikament zur parenteralen Verabreichung ist.

5. Verwendung nach Anspruch 1, wobei das Medikament zur Verabreichung in einer Konzentration im Bereich von etwa 1 µg/kg bis etwa 20.000 µg/kg formuliert ist.

6. Verwendung nach Anspruch 1, wobei das Medikament zur Behandlung einer Schädigung durch ein chemisches Toxin ist, das ausgewählt ist aus Ethanol, Blei, Cadmium, Kohlenstofftetrachlorid und Acetaminophen.

7. Verwendung nach Anspruch 1, wobei das Medikament zur Behandlung einer Schädigung durch eine bakterielle- oder virale Infektion ist.

8. Verwendung nach Anspruch 7, wobei die bakterielle- oder virale Infektion *durch einen* Organismus verursacht wird, der ausgewählt ist aus *Staphylococcus*-Spezies, *Streptococcus*-Spezies, *Neisseria*-Spezies, *Salmonella-* Spezies, *Shigella*-Spezies, *Escherichia coli, Clostridium perfringens, Klebsiella*-Spezies, *Proteus*-Spezies, *Enterobacter*-Spezies, *Bacteroides*-Spezies, *Brucella*-Spezies, *Francisella tularensis, Listeria monocytogenes, Acinetobacter*-Spezies, *Streptobacillus moniliformis, Vibrio*-Spezies, *Helicobacter pylori, Pseudomonas*-Spezies, *Haemophilus*-Spezies, *Bordetella pertussis*, Influenza-Viren, Adenoviren, Paramyxoviren, Rubellaviren, Polioviren, Hepatitisviren, Herpesviren, Rabiesviren, Menschliche Immunschwäche Viren (Human Immunodeficiency Viruses) und Papillomaviren.

9. Verwendung nach Anspruch 1, wobei sich das Deltorphin in einer pharmazeutisch verträglichen Formulierung befindet.

## Revendications

1. L'utilisation d'une deltorphine pour la fabrication d'un médicament destiné au traitement de lésion hépatique provoquée par la cytokine.

2. L'utilisation selon la revendication 1, dans laquelle ladite deltorphine est choisie dans le groupe constitué par deltorphine I SEQ ID NO : 1, deltorphine II SEQ ID NO : 2, et leurs combinaisons.

3. L'utilisation selon la revendication 1, ladite deltorphine étant sous une forme choisie dans le groupe constitué par une solution, une émulsion et suspension.

4. L'utilisation selon la revendication 1, ledit médicament étant destiné à l'administration parentérale.

5. L'utilisation selon la revendication 1, ledit médicament étant formulé pour l'administration à une concentration comprise entre environ 1 µg/kg et environ 20.000 µg/kg.

6. L'utilisation selon la revendication 1, ledit médicament étant destiné au traitement de lésion provoquée par une toxine chimique choisie dans le groupe constitué par l'éthanol, le plomb, le cadmium, le tétrachlorure de carbone et l'acétaminophène.

7. L'utilisation selon la revendication 1, ledit médicament étant destiné au traitement de lésion provoquée par une infection bactérienne ou virale.

8. L'utilisation selon la revendication 7, dans laquelle l'infection bactérienne ou virale est provoquée par un organisme choisi dans le groupe constitué par des espèces de staphylocoques, des espèces de streptocoques, des espèces de Neisseria, des espèces de Salmonella, des espèces de Shigella, Escherichia coli, Clostridium perfringens, des espèces de Klebsiella, des espèces de Proteus, des espèces d'Enterobacter, des espèces de Bactéroïdes, des espèces de Brucella, Francisella tularensis, Listeria monocytogènes, des espèces d'Acinetobacter, Streptobacillus monoiliformis, des espèces de Vibrio, Helicobacter pylori, des espèces de Pseudomonas, des espèces de Haemophilus, Bordetella pertussis, des virus de la grippe, des adénovirus, des paramyxovirus, des virus de la rubéole, des virus poliomyélitiques, des virus de l'hépatite, des virus de l'herpès, des virus de la rage, des virus de l'immunodéficience humaine et des virus du papillome.

9. L'utilisation selon la revendication 1, ladite deltorphine étant présentée dans une formulation pharmaceutiquement acceptable.
